# EUROPEAN PATENT APPLICATION

(11) **EP 4 282 824 A1**
(43) Date of publication of application: **29.11.2023**
(21) Application number: 21921515.9
(22) Date of filing: 18.10.2021
(51) Int. Cl.: C01F 7/47

(54) **METHOD OF HYDROCHEMICAL ENRICHMENT OF HIGH-CARBONATE BAUXITES FOR ALUMINA PRODUCTION**

(30) Priority: 21.01.2021 RU 2021101156
(71) Applicant: Obshchestvo s Ogranichennoy Otvetstvennost'yu "Obedinennaya Kompaniya Rusal Inzhenerno-Tekhnologicheskiy Tsentr", Krasnoyarsk 660111 (RU)
(72) Inventor: PECHENKIN, Maksim Nikolaevich, Krasnoyarsk, 660111 (RU); DAMASKIN, Aleksandr Aleksandrovich, Krasnoyarsk, 660111 (RU); DAMASKINA, Anna Aleksandrovna, Krasnoyarsk, 660111 (RU); ORDON, Sergej Fedorovich, Krasnoyarsk, 660111 (RU); PANOV, Andrej Vladimirovich, Krasnoyarsk, 660111 (RU)
(74) Representative: Atalay, Baris
(86) International application number: PCT/RU2021/050345
(87) International publication number: WO 2022/158999

(57) **Abstract**

The invention relates to a method of beneficiation of high-carbonated bauxites to remove carbonates to produce alumina by the Bayer process, said method comprising grinding bauxite in a solution of ethylenediaminetetraacetic acid (EDTA) disodium salt with a concentration of no less than 100 g/dm³, holding the obtained slurry while stirring at a temperature of no less than 70 °C, followed by filtering the obtained slurry to obtain a beneficiated bauxite and solution saturated with calcium salts, the beneficiated bauxite with a reduced carbonate content is fed to alumina recovery and the solution saturated with calcium salts is fed to regeneration of the EDTA disodium salt. The proposed method provides for effective removal of carbonates from bauxites (> 90 %), as well as increases the recovery of market-grade alumina from bauxites.

## Description

### Technical field

The invention relates to non-ferrous metallurgy, in particular to the production of alumina by the Bayer process, namely to the method of beneficiation of high-carbonated bauxites to reduce carbonate content.

### Background

There is a number of bauxite deposits where CO2 content in the ore reaches 5÷15 wt.%. Bauxites with such carbonate content were usually processed by an expensive and energy-consuming sintering method. Processing such bauxites by the Bayer method promotes a gradual accumulation of carbonate soda in process liquors due to the high carbonate content in the bauxites (up to 6 wt.% of CO2). A high soda content in the liquors results in a degradation of key performance indicators (hereinafter referred to as KPIs) of alumina production and adversely affects many key areas, namely, decreases the output and increases steam consumption in the digestion and evaporation areas, distorts the balance of alkali consumption by more extensive use of costly caustic soda.

A high input of carbonates with bauxite leads to the generation of a large amount of recycled soda that increases the moisture of the mixture fed to the sintering kilns, increases the specific fuel consumption per tonne of the sinter, and deteriorates the kiln performance.

The problem of carbonate removal from bauxites has been solved in a number of technical solutions.

The related art discloses a method for processing high-carbonated bauxites (patent SU No. 1276623, C01F7/30, publication date: December 15, 1986) that comprises preliminary calcining a part of high-carbonated bauxite ~ 16 wt.%, then combining the calcined bauxite with 84 % of the other bauxite and directing it to digestion.

The drawbacks of this method are as follows:
- necessity to perform the bauxite calcining, which is a capital-intensive and energy-consuming operation;
- removal of carbonates only from a part of the flow instead of the entire flow that reduces the effect;
- necessity to mix calcined and raw bauxites that requires additional infrastructure;
- probability of obtaining an excessively calcined material, recovery from which will be lower than the target values;
- incomplete decomposition of carbonate-containing minerals.

The related art discloses a method for obtaining alumina from bauxites with a high carbonate content (Paromova I.V. Research and development of a technology for alumina production from bauxites with a high carbonate content. A dissertation for a Degree in Technical Sciences, 1996). A technology was proposed for processing high-carbonated bauxites of the Krasnooktyabrskoe deposit comprising low-temperature calcining of high-sideritized bauxite at a temperature of 550-600 °C and exposure for 30 minutes. Under these conditions, siderite and kaolinite break down, organic impurities are completely removed from the bauxite, 80÷90 % of CO2 is removed, but calcite remains unaffected.

The drawbacks of this method, as in the previous patent, are as follows:
- necessity to perform the bauxite calcining, which is a capital-intensive and energy-consuming operation;
- calcining of the entire flow of bauxite thus increasing capital and operating costs;
- high probability of obtaining excessively calcined bauxite, recovery from which will be lower than the target values;
- incomplete decomposition of carbonate-containing minerals (calcite).

The related art discloses a method of bauxite processing (patent RU No. 1092142, C01F7/04, publication date: May 15, 1984) comprising flotation to separate low-quality (with a high carbonate content) and high-quality (with a low carbonate content) products, wherein the low-quality product is processed by sintering, and high-quality product is processed by the Bayer method. Flotation separation is carried out in an alkali-aluminate solution with Na2Oₜₒₜₐₗ concentration of 140÷320 g/dm³ and the caustic to alumina ratio of 3.5÷3.7.

The drawbacks of this method are as follows:
- necessity to purchase a large amount of reagents for flotation (grease and acid collectors, i.e. oleic acid, trans-fatty acids, tall acids, sulphanole), as well as flotation regulators, i.e. sodium silicate (water glass);
- ingress of a solution with a high concentration of caustic alkali to the sintering area that will reduce input of cheaper soda ash and increase the alumina production cost;
- ingress of organic flotation reagents to the Bayer liquors that will result in foaming and additional decausticization of circulating caustic alkali;
- dependence of the sintering area performance on the yield of the flotation tailings.

The method closest to the claimed one is a method of reducing the carbon dioxide content in bauxites by removing calcite from them using the flotation method (Farukshina Z.G., Kuznetsov V.P. On bauxite conditioning by calcium oxide. - In the book of Methodological materials for laboratories of geological organizations. Moscow, 1972, issue 3, pages 21-23 (VIMS). It can be used for processing diaspore-boehmite bauxites with a calcite content of over 2 %. The process includes bauxite grinding to the particle size of 0.44 mm, partial deslurring and flotation of calcite using oleic acid (0.1 kg/t) in the presence of OP-7 (OΠ-7) (0.05 kg/t), soda (0.4 kg/t) and sodium silicate (water glass) (0.2 kg/t). The bauxite concentrate comprises flotation tailings (basic and purification products) and muds. As a result of the beneficiation, the content of calcium oxide and carbon dioxide in the bauxite concentrate reduces by 2.8 times. The concentrate yield is 86-88 %.

The drawbacks of this method are as follows:
- the process involves several additional stages (deslurring, purification) and includes a stage of concentrate purification that increases the number of the required equipment;
- the use of several reagents for flotation that are not used in alumina production, which will get to the alumina production process with the concentrate, i.e. oleic acid, OP-7 (OΠ-7), sodium silicate (water glass);
- low concentrate yield, i.e. 86-88%.

### Disclosure of the invention

The objective of the present invention is to develop an economically viable method for removal of carbonates when processing high-carbonated bauxites to produce alumina; said method should allow obtaining bauxite with any given carbonate content. The bauxite beneficiated using said method is processed according to the existing alumina production process.

The technical effect lies in meeting the objective and efficient removal of carbonates from bauxites (> 90 %) as well as an increase in the output of smelter grade alumina.

The technical effect is achieved by a method of beneficiation of high-carbonated bauxites, said method comprising: mixing ethylenediaminetetraacetic acid disodium salt with bauxite, which selectively reacts with carbonate-containing minerals solubilizing them, wherein the amount of a solution of ethylenediaminetetraacetic acid disodium salt to dissolve carbonates is calculated by stoichiometry, grinding of the bauxite is carried out and then the obtained bauxite mixture is held at a temperature of preferably no less than 70 °C while stirring to reduce the carbonate concentration in the bauxite to less than 0.5 wt.%, the slurry obtained after the holding is filtered, the beneficiated bauxite with a reduced carbonate content is fed to alumina recovery, and the solution saturated with calcium salts is fed to regeneration of ethylenediaminetetraacetic acid disodium salt.

Alternatively, the method for beneficiation of high-carbonated bauxites includes mixing EDTA (ethylenediaminetetraacetic acid) disodium salt with a preferable concentration of 100-230 g/dm³ with the bauxite, grinding the solid phase to obtain no less than 75 % of 0.056 mm fraction, holding at a temperature of preferably no less than 70 °C, in most cases while stirring for preferably no less than 1 hour, then the obtained slurry undergoes filtration to obtain beneficiated bauxite and a solution saturated with calcium salts, the beneficiated bauxite with a reduced carbonate content is fed to mixing with raw bauxite at a weight ratio preferably ranging from 40/60 to 50/50, and the obtained mixture is directed to alumina recovery.

The method is supplemented by its embodiments.

In the particular embodiment of the invention, an amount of the EDTA disodium salt solution for removal of carbonates is calculated by stoichiometry for complete removal of carbonates. Lime is added to the mixture of beneficiated and raw bauxites fed to alumina recovery to obtain CaO content of 5 %.

After the slurry filtration, the solution is fed to regeneration of EDTA disodium salt and returned to the head of the beneficiation process. EDTA disodium salt is regenerated using crystalline oxalic acid.

The saturated EDTA calcium solution is treated with crystalline oxalic acid at a temperature of 60-90 °C and residence time of no less than 10 minutes to form EDTA disodium salt and insoluble calcium oxalate.

The obtained slurry is filtered and after the slurry filtration, the EDTA disodium salt solution is returned to the beneficiation of high-carbonated bauxite. After the slurry filtration, the cake is fed to regeneration of oxalic acid from calcium oxalate.

Oxalic acid is regenerated by a sulfuric acid solution at a temperature of no less than 60 °C and residence time of no less than 10 minutes to form a mixture of sulfuric and oxalic acids and insoluble calcium oxalate.

The obtained slurry is filtered and after the slurry filtration, the solid phase, represented by gypsum is dumped to a disposal area or used for production of construction materials.

After the slurry filtration, the solution, represented by a mixture of oxalic and sulfuric acids, is fed to crystallization of oxalic acid. The crystallization of oxalic acid is carried out by cooling down to a temperature of 10-25 °C.

The cooled slurry is filtered and after the filtration of the cooled slurry, crystalline oxalic acid is fed to regeneration of EDTA disodium salt. After the filtration of the cooled slurry, the sulfuric acid solution is adjusted with a fresh acid with the concentration of 98 wt.% and is fed to regeneration of oxalic acid.

### Brief Description of the Drawings

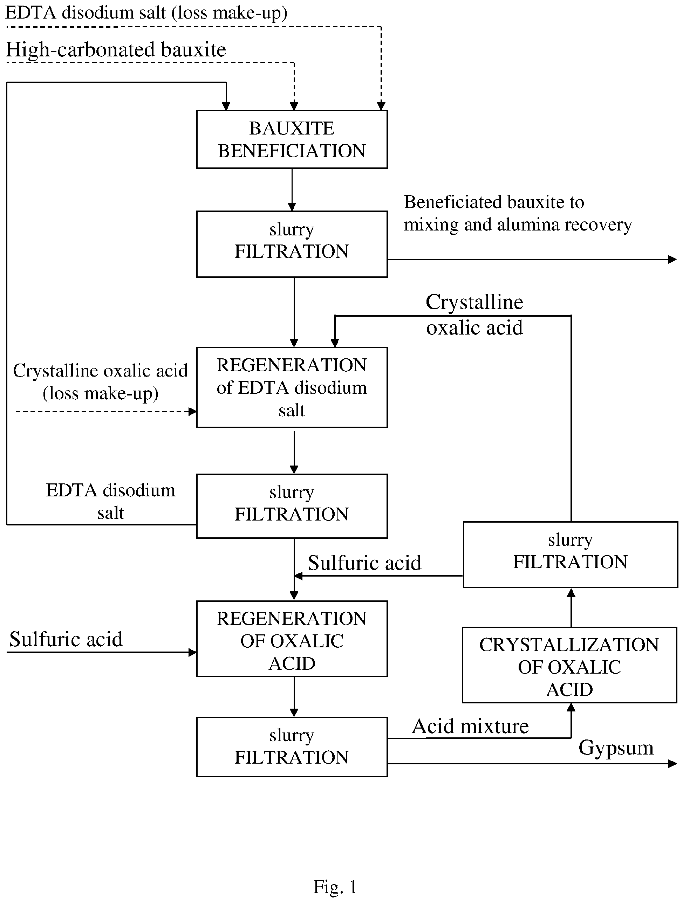

Fig. 1 shows a process flow diagram of the beneficiation of high-carbonated bauxites to reduce carbonate content.

### Detailed Description of the Embodiment

The method is intended for beneficiation of high-carbonated bauxites containing a large amount of carbonate impurities (5÷15 wt.% of CO2) in the presence of ethylenediaminetetraacetic acid (EDTA) disodium salt that selectively reacts with carbonate-containing minerals solubilizing them. The amount of the EDTA disodium salt solution for removal of carbonates is calculated by stoichiometry for the complete removal of carbonates. The EDTA disodium salt solution with a concentration of 100÷230 g/dm³ is mixed with bauxite directly in a ball mill. Grinding in a mill is carried out up to the size of the bauxite solid phase of no less than 75 % of 0.056 mm fraction. To ensure the completeness of the reaction, bauxite grinding shall be followed by the holding at a temperature of no less than 70 °C while stirring for no less than 1 hour. Following the holding, the carbonate concentration in bauxite reduces to less than 0.5 wt.%.The obtained slurry is filtered, the beneficiated bauxite (filter cake) with a reduced carbonate content is fed to mixing with raw bauxite at a weight ratio ranging from 40/60 to 50/50. The obtained mixture is fed to alumina recovery by the existing process, and the solution saturated with calcium salts is fed to regeneration of EDTA disodium salt.

The saturated EDTA calcium solution is treated with crystalline oxalic acid at a temperature of 60-90 °C and residence time of no less than 10 minutes. As a result of the reaction, EDTA disodium salt and insoluble calcium oxalate are formed. The obtained slurry is filtered, the EDTA disodium salt solution is recycled to beneficiation of high-carbonated bauxite, and the residue is fed to regeneration of oxalic acid from calcium oxalate. Oxalic acid is regenerated by a sulfuric acid solution at a temperature of no less than 60 °C and residence time of no less than 10 minutes. As a result of the reaction, a mixture of sulfuric and oxalic acids and insoluble calcium oxalate are formed. The obtained slurry is filtered, the solid phase is gypsum that is either dumped to a disposal area or used for production of construction materials. The solution, represented by a mixture of oxalic and sulfuric acids is fed to crystallization of oxalic acid with cooling down to a temperature of 10-25 °C. The cooled slurry is filtered, crystalline oxalic acid is fed to regeneration of EDTA disodium salt, and the sulfuric acid solution is adjusted using a fresh acid with the concentration of 98 wt.%, and is fed to regeneration of oxalic acid.

Thus, the proposed method of hydrochemical beneficiation of high-carbonated bauxites ensures a high degree of carbonate removal by means of using a solution of EDTA disodium salt with its subsequent regeneration and return to the head of the process for beneficiation.

*The following examples illustrate the proposed method.*

### Example 1

SUBR high-carbonated bauxite, GB-1 grade, with CO2 content of 6.0 wt.%, was grinded to obtain no less than 75 % of 0.056 fraction with a solution of EDTA disodium salt with the concentration of 100 g/dm³ in the amount calculated by stoichiometry to provide for the carbonate removal. The obtained slurry was held for 2 hours at a temperature of 90 °C. After the holding, the slurry was filtered, the solid phase, i.e. beneficiated bauxite, contained 0.39 wt.% of CO2, carbonate recovery amounted to 94.3 wt.%. Alumina recovery from digesting a mixture of beneficiated and raw bauxites at the weight ratio of 45/55 amounted to 90.6 wt.%., alumina recovery from digesting raw bauxite amounted to 88.6 wt.%.

### Example 2 (based on example 1)

SUBR high-carbonated bauxite, GB-1 grade, with CO2 content of 6.0 wt.%, was grinded to obtain no less than 75 % of 0.056 fraction with a solution of EDTA disodium salt with the concentration of 230 g/dm³ in the amount calculated by stoichiometry to provide for the carbonate removal. The obtained slurry was held for 2 hours at a temperature of 95 °C. After the holding, the slurry was filtered, the solid phase, i.e. beneficiated bauxite, contained 0.40 wt.% of CO2, carbonate recovery amounted to 94.2 wt.%. Alumina recovery from digesting a mixture of beneficiated and raw bauxites at the weight ratio of 45/55, with an addition of burnt lime up to the content of 5% CaO in the mixture, amounted to 91.3 wt/%, alumina recovery from digesting raw bauxite amounted to 88.6 wt.%.

### Example 3 (based on example 2)

SUBR high-carbonated bauxite, GB-2 grade, with CO2 content of 15.0 wt.%, was grinded to obtain no less than 75 % of 0.056 fraction with a solution of EDTA disodium salt with the concentration of 230 g/dm³ in the amount calculated by stoichiometry to provide for the carbonate removal. The obtained slurry was held for 1 hour at a temperature of 95 °C. After the holding, the slurry was filtered, the solid phase, i.e. beneficiated bauxite, contained 0.77 wt.% of CO2, carbonate recovery amounted to 96.1 wt.%. Alumina recovery from digesting a mixture of beneficiated and raw bauxites at the weight ratio of 40/60 amounted to 88.9 wt.%, alumina recovery from digesting raw bauxite amounted to 88.6 wt.%.

### Example 4 (based on example 2)

SUBR high-carbonated bauxite, GB-1 grade, with CO2 content of 6.0 wt.%, was grinded to obtain no less than 75 % of 0.056 fraction with a solution of EDTA disodium salt with the concentration of 230 g/dm³ in the amount calculated by stoichiometry to provide for the carbonate removal. The obtained slurry was held for 3 hours at a temperature of 70 °C. After the holding, the slurry was filtered, the solid phase, i.e. beneficiated bauxite, contained 0.45 wt.% of CO2, carbonate recovery amounted to 93.4 wt.%. Alumina recovery from digesting a mixture of beneficiated and raw bauxites at the weight ratio of 50/50 amounted to 88.9 wt.%, alumina recovery from digesting raw bauxite amounted to 88.6 wt.%.

### Example 5 (based on example 2)

SUBR high-carbonated bauxite, GB-1 grade, with CO2 content of 6.0 wt.%, was grinded to obtain no less than 75 % of 0.056 fraction with a solution of EDTA disodium salt with the concentration of 230 g/dm³ in the amount calculated by stoichiometry to provide for the carbonate removal. The obtained slurry was held for 3.0 hours at a temperature of 95 °C. After the holding, the slurry was filtered, the solid phase, i.e. beneficiated bauxite, contained 0.38 wt.% of CO2, carbonate recovery amounted to 94.5 wt.%. Alumina recovery from digesting a mixture of beneficiated and raw bauxites at the weight ratio of 50/50 amounted to 89.1 wt.%, alumina recovery from digesting raw bauxite amounted to 88.6 wt/%.

Thus, the proposed method of hydrochemical beneficiation of high-carbonated bauxites containing up to 15 wt.% CO2, using EDTA disodium salt as a carbonate removing reagent ensures not only effective removal of carbonate-containing minerals but also increase in the alumina recovery from bauxites. Therefore, the alumina recovery from a mixture of beneficiated and raw bauxites is 0.3÷2 % higher than from raw bauxite.

A protective right is claimed for the following scope.
1. A method of beneficiation of high-carbonated bauxites said method comprising:
   - mixing an ethylenediaminetetraacetic acid (EDTA) disodium salt with a bauxite, said salt selectively reacting with carbonate-containing minerals solubilizing the carbonate-containing minerals, wherein the amount of a solution of the ethylenediaminetetraacetic acid disodium salt required to dissolve carbonates is calculated by stoichiometry,
   - grinding the bauxite in the resulted mixture,
   - holding [the obtained bauxite mixture] at a temperature of preferably no less than 70 °C while stirring to reduce the carbonate concentration in the bauxite to less than 0.5 wt.%,
      wherein a slurry obtained from the holding is filtered, a beneficiated bauxite with a reduced carbonate content is directed to alumina recovery, and a solution saturated with calcium salts is fed to a regeneration of the ethylenediaminetetraacetic acid disodium salt.
2. The method of claim 1, wherein the EDTA disodium salt is used with a concentration of preferably no less than 100 g/dm³, and the holding and stirring are carried out at a temperature of no less than 70 °C.
3. The method of claim 1, wherein the amount of the solution of the EDTA disodium salt for removal of the carbonates is calculated by stoichiometry to achieve a complete removal of the carbonates.
4. The method of claim 1, wherein lime is added to a mixture of beneficiated and raw bauxites fed to alumina recovery to achieve a CaO content of 5 %.
5. The method of claim 1, wherein lime is added to the beneficiated bauxite fed to alumina recovery to achieve a CaO content of 5 %.
6. The method of claim 1, wherein the solution obtained from the slurry filtration is fed to the regeneration of the EDTA disodium salt and returned to the head of the beneficiation process.
7. The method of claim 1, wherein the regeneration of the EDTA disodium salt is carried out using a crystalline oxalic acid.
8. The method of claim 7, wherein the saturated EDTA calcium solution is treated with the crystalline oxalic acid at a temperature of 60-90 °C and residence time of no less than 10 minutes to form the EDTA disodium salt and an insoluble calcium oxalate.
9. The method of claim 8, wherein the obtained slurry undergoes filtration.
10. The method of claim 8, wherein after the slurry filtration the solution of the EDTA disodium salt is recycled to the beneficiation of the high-carbonated bauxite.
11. The method of claim 8, wherein after the slurry filtration a cake is fed to a regeneration of the oxalic acid from the calcium oxalate.
12. The method of claim 11, wherein the regeneration of the oxalic acid is carried out with a sulfuric acid solution at a temperature of no less than 60 °C and residence time of no less than 10 minutes to form a mixture of solutions of sulfuric and oxalic acids and the insoluble calcium oxalate.
13. The method of claim 12, wherein the obtained slurry undergoes filtration.
14. The method of claim 13, wherein after the slurry filtration a solid phase represented by gypsum is either dumped to a disposal area or used for a production of construction materials.
15. The method of claim 13, wherein after the slurry filtration the solution being a mixture of the oxalic and sulfuric acids is fed to a crystallization of the oxalic acid.
16. The method of claim 15, wherein the crystallization of the oxalic acid is carried out with cooling down to a temperature of 10-25 °C.
17. The method of claim 15, wherein the cooled slurry undergoes filtration.
18. The method of claim 17, wherein after the filtration of the cooled slurry, the crystalline oxalic acid is fed to the regeneration of the EDTA disodium salt.
19. The method of claim 17, wherein after the filtration of the cooled slurry, the sulfuric acid solution is adjusted with a fresh acid with a concentration of 98 wt.% and is fed to the regeneration of the oxalic acid.

## Claims

1. A method of beneficiation of high-carbonated bauxites, said method comprising:
- mixing an ethylenediaminetetraacetic acid (EDTA) disodium salt with a bauxite, said salt selectively reacting with carbonate-containing minerals solubilizing the carbonate-containing minerals, wherein the amount of a solution of the ethylenediaminetetraacetic acid disodium salt required to dissolve carbonates is calculated by stoichiometry,
- grinding the bauxite in the resulted mixture,
- holding [the obtained bauxite mixture] at a temperature of preferably no less than 70 °C while stirring to reduce the carbonate concentration in the bauxite to less than 0.5 wt.%,
wherein a slurry obtained from the holding is filtered, a beneficiated bauxite with a reduced carbonate content is directed to alumina recovery, and a solution saturated with calcium salts is fed to a regeneration of the ethylenediaminetetraacetic acid disodium salt.

2. The method of claim 1, wherein the ethylenediaminetetraacetic acid disodium salt is used with a concentration of preferably no less than 100 g/dm³, and the holding and stirring are carried out at a temperature of no less than 70°C.

3. The method of claim 1, wherein the amount of the solution of the EDTA disodium salt for removal of the carbonates is calculated by stoichiometry to achieve a complete removal of the carbonates.

4. The method of claim 1, wherein lime is added to a mixture of beneficiated and raw bauxites fed to alumina recovery to achieve a CaO content of 5 %.

5. The method of claim 1, wherein lime is added to the beneficiated bauxite fed to alumina recovery to achieve a CaO content of 5 %.

6. The method of claim 1, wherein the solution obtained from the slurry filtration is fed to the regeneration of the EDTA disodium salt and returned to the head of the beneficiation process.

7. The method of claim 1, wherein the regeneration of the EDTA disodium salt is carried out using a crystalline oxalic acid.

8. The method of claim 7, wherein the saturated EDTA calcium solution is treated with the crystalline oxalic acid at a temperature of 60-90 °C and residence time of no less than 10 minutes to form the EDTA disodium salt and an insoluble calcium oxalate.

9. The method of claim 8, wherein the obtained slurry undergoes filtration.

10. The method of claim 8, wherein after the slurry filtration the solution of the EDTA disodium salt is recycled to the beneficiation of the high-carbonated bauxite.

11. The method of claim 8, wherein after the slurry filtration a cake is fed to a regeneration of the oxalic acid from the calcium oxalate.

12. The method of claim 11, wherein the regeneration of the oxalic acid is carried out with a sulfuric acid solution at a temperature of no less than 60 °C and residence time of no less than 10 minutes to form a mixture of solutions of sulfuric and oxalic acids and the insoluble calcium oxalate.

13. The method of claim 12, wherein the obtained slurry undergoes filtration.

14. The method of claim 13, wherein after the slurry filtration a solid phase represented by gypsum is either dumped to a disposal area or used for a production of construction materials.

15. The method of claim 13, wherein after the slurry filtration the solution being a mixture of the oxalic and sulfuric acids is fed to a crystallization of the oxalic acid.

16. The method of claim 15, wherein the crystallization of the oxalic acid is carried out with cooling down to a temperature of 10-25 °C.

17. The method of claim 15, wherein the cooled slurry undergoes filtration.

18. The method of claim 17, wherein after the filtration of the cooled slurry, the crystalline oxalic acid is fed to the regeneration of the EDTA disodium salt.

19. The method of claim 17, wherein after the filtration of the cooled slurry, the sulfuric acid solution is adjusted with a fresh acid with a concentration of 98 wt.% and is fed to the regeneration of the oxalic acid.
